# EUROPEAN PATENT APPLICATION

(11) **EP 4 488 390 A1**
(43) Date of publication of application: **08.01.2025**
(21) Application number: 23382703.9
(22) Date of filing: 07.07.2023
(51) Int. Cl.: C12Q 1/6886

(54) **IN VITRO METHOD FOR THE PROGNOSIS OF PATIENTS SUFFERING FROM TRIPLE-NEGATIVE BREAST CANCER**

(71) Applicant: Reveal Genomics S.L., 08036 Barcelona (ES)
(72) Inventor: PRAT APARICIO, Aleix, 08036 Barcelona (ES); BRASÓ MARISTANY, Fara, 08036 Barcelona (ES); PARÉ BRUNET, Laia, 08036 Barcelona (ES)
(74) Representative: Hoffmann Eitle

(57) **Abstract**

The present invention refers to an *in vitro* method for the prognosis of patients suffering from triple-negative breast cancer (TNBC).

## Description

### FIELD OF THE INVENTION

The present invention refers to the medical field. Particularly, the present invention refers to an *in vitro* method for the prognosis of patients suffering from triple-negative breast cancer (TNBC).

### STATE OF THE ART

The implementation of biomarkers to guide therapy is crucial for the de-escalation and escalation of systemic therapy in early-stage TNBC. Currently, the identification of the key genes responsible for driving this disease, specifically the core immune TNBC genes, remains unknown. Additionally, it is unclear whether combinations of these genes can enhance their association with clinical endpoints.

To effectively optimize treatment strategies, it is essential to determine which specific immune genes play a central role in TNBC. By identifying the core immune TNBC genes, researchers can gain valuable insights into the underlying molecular mechanisms driving the disease's progression and treatment response. This knowledge will enable the development of more targeted therapies that focus on modulating these key genes and their associated pathways.

Furthermore, investigating the potential synergy between different immune genes may offer additional therapeutic opportunities. By studying combinations of these genes, researchers can explore whether their collective expression has a stronger association with clinical endpoints than individual gene expression. This approach could provide a more comprehensive understanding of the complex immune response in TNBC and potentially lead to the development of novel therapeutic strategies.

In summary, there is an unmet medical need of finding new tools focused on the prognosis of patients suffering from TNBC. The de-escalation and escalation of systemic therapy in early-stage TNBC require the integration of biomarkers to guide treatment decisions. It remains essential to identify the core immune TNBC genes responsible for driving the disease. Additionally, exploring combinations of these genes may enhance their association with clinical endpoints and unlock new therapeutic possibilities. Ultimately, this research will contribute to more precise and personalized approaches for the management of early-stage TNBC. The present invention is precisely focused on solving this problem and it is herein provided a new tool for the prognosis of patients suffering from TNBC which helps the clinicians to design or elect therapies in this particular type of cancer.

### DESCRIPTION OF THE INVENTION

### Brief description of the invention

Such as it is indicated above, de-escalation and escalation of systemic therapy in early-stage TNBC will require the implementation of biomarkers to guide therapy. To date, it is unknown which are they key genes driving this disease (i.e., the Core Immune Genes [CIG]) and if combinations of these genes can improve their association with both clinical endpoints.

Consequently, the inventors of the present invention evaluated the expression of up to 185 genes on pre-treatment tumor biopsies from 3 independent publicly available datasets of early-stage TNBC: CALGB40603 (n=389) (NCT00861705), BrighTNess (n=482) (NCT02032277), and SCAN-B (n=498). Patients in CALGB40603 and BrighTNess were treated with neoadjuvant chemotherapy-based therapy and had pathological complete response (pCR) data. Patients in CALGB40603 and SCAN-B had survival outcome data (event-free survival [EFS] or relapse-free interval [RFi]). Each individual gene was associated with each clinical endpoint within each dataset using Cox model and logistic regression analyses, adjusting for treatment arm. Genes found in common across datasets were selected as CIG, except for *PDCD1*, which was not available in BrighTNess. Using the CIGs, it was evaluated whether combinations of 2 genes enhance their association with each clinical endpoint using different methods: adding, subtracting, multiplying, and ratio. Likelihood ratio testing was used to evaluate the added contribution of each gene in 2 combined cohorts evaluating pCR (i.e., CALGB40603 and BrighTNess) and survival (i.e., CALGB40603 and SCAN-B).

Ten CIGs related to B-cells and T-cells (i.e., *IRF4, LAX1, POU2AF1, CD274, CD79A, TNFRSF17, PIM2, PDCD1, CXCR6,* and *SLAMF1*) were associated with higher pCR rates and improved survival outcome across datasets. The correlation coefficient among the 10 CIG genes was 0.63 (moderate). Such as it is shown, for instance, in **Table 6,** the sum of 2 CIGs provided more prognostic information than a single gene (p<0.001). Two-gene subtraction, multiplication or ratio decreased the amount of prognostic information (p<0.001). The top 10 2-gene CIGs associated with prognosis (p<0.001) were *CD274_TNFRSF17, IRF4_CD274, PDCD1_TNFRSF17, CD79A_CD274, CD274_POU2AF1, CXCR6_TNFRSF17, LAX1*_*TNFRSF17, SLAMF1*_*TNFRSF17, CD274_LAX1 and CD79A_CXCR6* (**Table 6**). On the other hand, such as it is shown, for instance, in **Table 7,** the sum of 2 CIGs provided more information about pCR than a single gene (p<0.001). Two-gene subtraction, multiplication or ratio decreased the amount of information about pCR (p<0.001). The top 10 2-gene CIGs were *CD274_PIM2, CD274_POU2AF1, CXCR6_PIM2, CD274_CXCR6, IRF4_CD274, CXCR6_POU2AF1, CD274_LAX1, IRF4_CXCR6, CXCR6_LAX1,* and *POU2AF1_SLAMF1* (**Table 7**).

So, it is herein shown that the expression of CIGs associated with B-cell and T-cell adaptive immune response is closely linked to pCR and survival outcomes following (neo)adjuvant chemotherapy. Particularly, the combination of the expression level of at least two genes selected from the list: *IRF4, LAX1, POU2AF1, CD274, CD79A, TNFRSF17, PIM2, PDCD1, CXCR6,* and *SLAMF,* for instance any combination of the expression level of two, three, four, five, six, seven, eight, nine or ten of the genes: *IRF4, LAX1, POU2AF1, CD274, CD79A, TNFRSF17, PIM2, PDCD1, CXCR6,* and *SLAMF1* provides more informative insights into pCR and survival outcomes compared to other combination methods. This approach enhances the predictive power of CIGs, enabling a better understanding of treatment response and patient prognosis, ultimately contributing to improved clinical decision-making and patient outcomes.

Thus, the present invention demonstrates a strong association between the expression of CIGs related to B-cell and T-cell adaptive immune response and the achievement of pCR as well as survival outcomes in patients who underwent (neo)adjuvant chemotherapy for TNBC. These findings highlight the crucial role of the immune system in determining treatment response and long-term prognosis in early-stage TNBC. The expression levels of these CIGs can serve as reliable biomarkers to assess the likelihood of achieving pCR and predict survival outcomes in TNBC patients.

Overall, the novelty of adding the expression of 2 immune genes for outcome prediction lies in capturing synergistic immune responses, considering multi-dimensional immune activity, addressing immune response heterogeneity, uncovering gene interactions, and offering potential clinical applications. By moving beyond single-gene approaches, this novel strategy provides a more comprehensive and nuanced understanding of immune functionality and its relationship to outcomes in various disease contexts.

In conclusion, our study highlights the significance of CIGs associated with B-cell and T-cell adaptive immune response in early-stage TNBC. Through the combination of at least 2 specific CIGs using an additive approach, we observed an improved ability to predict pCR and survival outcomes in TNBC patients. These findings contribute to a better understanding of the complex interplay between the immune system and TNBC, ultimately aiding in the development of personalized treatment strategies. By harnessing the predictive power of CIGs, healthcare professionals can make more informed clinical decisions, leading to improved patient outcomes in the management of TNBC.

So, the first embodiment of the present invention refers to an *in vitro* method for identifying biomarker signatures for the prognosis of patients suffering from TNBC, which comprises: a) Measuring the level of expression of at least two genes selected from the group consisting of: *IRF4, LAX1, POU2AF1, CD274, CD79A, TNFRSF17, PIM2, PDCD1, CXCR6* and/or *SLAMF1*, in a biological sample obtained from the patient; b) determining a combination score value by calculating the sum of the expression of the two genes; and c) wherein if a deviation of the combination score value is identified, as compared with a pre-established reference value, this is indicative that the biomarker signature may be used for the prognosis of patients suffering from TNBC.

The second embodiment of the present invention refers to an *in vitro* method for the prognosis of patients suffering from TNBC, which comprises: a) Measuring the level of expression of at least two genes selected from the group consisting of: *IRF4, LAX1, POU2AF1, CD274, CD79A, TNFRSF17, PIM2, PDCD1, CXCR6* and/or *SLAMF1*, in a biological sample obtained from the patient; b) determining a combination score value by calculating the sum of the expression of the two genes; and c) wherein if a deviation of the combination score value is identified, as compared with a pre-established reference value, this is indicative of the prognosis of patients suffering from TNBC.

In a preferred embodiment of the invention, the prognosis of patients suffering from TNBC comprises: a) Measuring the level of expression of at least one of the combinations of two genes of **Table 6** or **Table 7** in a biological sample obtained from the patient; b) determining a combination score value by calculating the sum of the expression of the two genes; and c) wherein if a deviation of the combination score value is identified, as compared with a pre-established reference value, this is indicative of the prognosis of patients suffering from TNBC.

In a preferred embodiment of the invention, the prognosis of patients suffering from TNBC comprises: a) Measuring the level of expression of at least two genes selected from the group consisting of: *IRF4, LAX1, POU2AF1, CD274, CD79A, TNFRSF17, PIM2, PDCD1, CXCR6* and/or *SLAMF1*, in a biological sample obtained from the patient; b) determining a combination score value by calculating the sum of the expression of the two genes; and c) wherein if an increased score value is identified, as compared with a pre-established reference value, this is indicative of good prognosis.

In a preferred embodiment of the invention, the prognosis of patients suffering from TNBC comprises: a) Measuring the level of expression of at least one of the combinations of two genes of **Table 6** or **Table 7** in a biological sample obtained from the patient; b) determining a combination score value by calculating the sum of the expression of the two genes; and c) wherein if an increased score value is identified, as compared with a pre-established reference value, this is indicative of good prognosis.

In a preferred embodiment of the invention, the prognosis of patients suffering from TNBC comprises: a) Measuring the level of expression of at least one of the combinations of two genes of **Table 6** in a biological sample obtained from the patient; b) determining a combination score value by calculating the sum of the expression of the two genes; and c) wherein if an increased score value is identified, as compared with a pre-established reference value, this is indicative of lack of cancer recurrence.

In a preferred embodiment of the invention, the prognosis of patients suffering from TNBC comprises: a) Measuring the level of expression of at least one of the combinations of two genes of **Table 7** in a biological sample obtained from the patient; b) determining a combination score value by calculating the sum of the expression of the two genes; and c) wherein if an increased score value is identified, as compared with a pre-established reference value, this is indicative of response to chemotherapy-based therapy.

In a preferred embodiment the biological sample is a tumor biopsy.

The second embodiment of the present invention refers to the *in vitro* use of at least two genes selected from the group consisting of: *IRF4, LAX1, POU2AF1, CD274, CD79A, TNFRSF17, PIM2, PDCD1, CXCR6* and/or *SLAMF1*, or of a kit comprising reagents for the assessing the level of expression of the genes, for identifying biomarker signatures for the prognosis of patients suffering from TNBC.

In a preferred embodiment the present invention refers to the *in vitro* use at least two genes selected from the group consisting of: *IRF4, LAX1, POU2AF1, CD274, CD79A, TNFRSF17, PIM2, PDCD1, CXCR6* and/or *SLAMF1*, or of a kit comprising reagents for the assessing the level of expression of the genes, for the prognosis of patients suffering from TNBC.

In a preferred embodiment the present invention refers to the *in vitro* use of at least one of the combinations of two genes of **Table 6** or **Table 7,** or of a kit comprising reagents for the assessing the level of expression of the genes, for the prognosis of patients suffering from TNBC.

In a preferred embodiment the present invention refers to the *in vitro* use of at least one of the combinations of two genes of **Table 6**, or of a kit comprising reagents for the assessing the level of expression of the genes, for predicting recurrence.

In a preferred embodiment the present invention refers to the *in vitro* use of at least one of the combinations of two genes of **Table 7,** or of a kit comprising reagents for the assessing the level of expression of the genes, for predicting response to chemotherapy-based therapy.

The third embodiment of the present invention refers to chemotherapy, PARP1 inhibitors, antibody drug conjugates and PD-1 or PD-L1 inhibitors for use in the treatment of patients suffering from TNBC, wherein the method comprises assessing the prognosis of the patients suffering from TNBC, by following any of the above methods of the invention. In this sense, the present invention also refers to a method for treating a patient suffering from TNBC which comprises the administration of a therapeutically effective dose or amount of chemotherapy, PARP1 inhibitors, antibody drug conjugates and PD-1 or PD-L1 inhibitors, once the prognosis of the patient has been evaluated following any of the above-cited methods.

In a preferred embodiment, the chemotherapy, PARP1 inhibitors, antibody drug conjugates and PD-1 or PD-L1 inhibitor is selected from the list comprising: Paclitaxel, Nab-paclitaxel, Docetaxel, Carboplatin, Cisplatinum, Cyclophosphamide, Doxorubicin, Olaparib, Niraparib, Talazoparib, Nivolumab, Pembrolizumab, Atezolizumab, Avelumab, Durvalumab, Cemiplimab, Tislelizumab, Dostarlimab or Retifanlimab, trastuzumab deruxtecan, sacitumab govitecan and patritumab deruxtecan.

The present invention also refers to the following the topics included below.

A method for detecting biomarkers in a test sample from a human subject suffering from TNBC, the method comprising: a) Contacting the test sample with a primer specific to at least two genes selected from the list: *IRF4, LAX1, POU2AF1, CD274, CD79A, TNFRSF17, PIM2, PDCD1, CXCR6* and/or *SLAMF1*; b) amplifying to produce an amplification product in the test sample; and, c) measuring the expression level by determining the level of the amplification product in the test sample.

In a preferred embodiment, the present invention is a computer-implemented invention, wherein a processing unit (hardware) and a software are configured to:
a) Receive expression level values of the genes *IRF4, LAX1, POU2AF1, CD274, CD79A, TNFRSF17, PIM2, PDCD1, CXCR6* and/or *SLAMF1*,
b) Process the expression level values received for finding substantial variations or deviations, and
c) Provide an output through a terminal display of the variation or deviation of the expression level, wherein the variation or deviation of the expression level indicates whether the patient has a good or bad prognosis.

Finally, the present invention also refers to a computer program or a computer-readable media containing means for carrying out a method defined above.

For the purpose of the present invention the following terms are defined:
- The term "reference level of expression measured", when referring to the level of the biomarkers described in the present invention, refers to expression of the CIGs normalized by the geometric mean level of 5 housekeeping genes (*ACTB, GAPD, PSMC4, PUM1*, and *RPLP0*). A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data.
- By the term "comprising" is meant the inclusion, without limitation, of whatever follows the word "comprising". Thus, use of the term "comprising" indicates that the listed elements are required or mandatory, but that other elements are optional and may or may not be present.
- By "consisting of" is meant the inclusion, with limitation to whatever follows the phrase "consisting of". Thus, the phrase "consisting of" indicates that the listed elements are required or mandatory, and that no other elements may be present.
- By "therapeutically effective dose or amount" of a composition is intended an amount that, when administered as described herein, brings about a positive therapeutic response in a subject having TNBC. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

**Figu**r**e 1**. Identification of CIGs associated with low risk and high probability of achieving a pCR in the CALGB40603, SCAN-B and BrighTNess TNBC datasets.

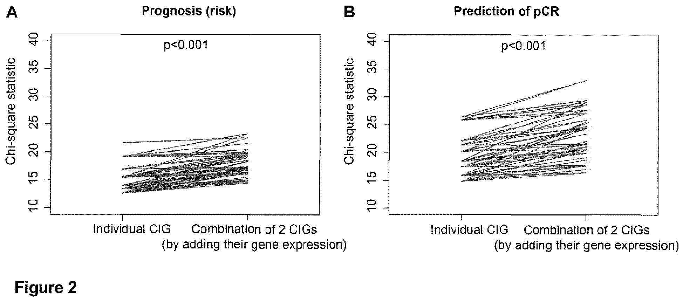

**Figure 2****.** Amount of variation explained in prognosis (A) and prediction of pCR (B) defined by chi-square (χ2) statistics from likelihood ratio tests.

### Detailed description of the invention

The present invention is specifically illustrated by means of the following examples without the intention of limiting the scope of protection to what it is described therein.

### Example 1. Methods

### Example 1.1. CALGB40603 cohort

The CALGB40603 phase II trial (NCT00861705) was a randomized study of paclitaxel (80 mg/m² once a week for 12 weeks) with or without carboplatin (area under the curve 6 once every 3 weeks for four doses) and/or bevacizumab (10 mg/kg once every 2 weeks for 9 doses) followed by dose-dense doxorubicin and cyclophosphamide in 443 patients with operable TNBC(1). Event-free survival (EFS) and pCR and RNA sequencing data (dbGaP accession number phs001863.v1.p1) were available for 389 patients.

### Example 1.2. BrighTNess cohort

The BrighTNess phase II trial (NCT02032277) evaluated the safety and efficacy of the addition of veliparib plus carboplatin versus the addition of carboplatin to standard neoadjuvant chemotherapy versus standard neoadjuvant chemotherapy in 634 patients with early stage TNBC(2, 3). Patients were randomized (2:1:1) to paclitaxel (80 mg/m² once a week for 12 weeks) concurrently with: (i) carboplatin (area under the curve 6 mg/ml/min, every 3 weeks, for four cycles) plus veliparib (50 mg orally twice daily); (ii) carboplatin plus veliparib placebo; or (iii) carboplatin placebo plus veliparib placebo. pCR and RNA sequencing data were available for 482 patients (GEO repository accession number GSE164458).

### Example 1.3. SCAN-B

The SCAN-B (Swedish Cancerome Analysis Network - Breast) is a research project and cohort study in Sweden focused on breast cancer consisting of 11,790 patients that provided an informed consent based on either a diagnosis of breast cancer or a suspected breast cancer. The project involves genomic sequencing of the tumors to identify specific genetic alterations and molecular markers associated with different subtypes of breast cancer(4). Relapse-free interval (RFi) data and RNA sequencing data from the last updated version of the SCAN-B dataset was retrieved for 498 patients with TNBC from Mendeley Data (https://data.mendeley.com/datasets/yzxtxn4nmd).

### Example 1.4. Statistical analyses

The first objective was to identify the association of up to 185 genes **(Table 1),** including immune-related and breast cancer-related genes (such as genes of the PAM50 and HER2DX assays), with prognosis and prediction of pCR. Of the 185 gene list, 182 (98.4%), 180 (97.3%) and 177 (96.0%) were available from CALGB40603, SCAN-B and BrighTNess, respectively. Univariate and multivariable Cox regression models and logistic regression models adjusting for treatment arm were used to investigate the association for individual gene in terms of hazard ratios (HRs) or odds-ratios (ORs) with 95% confidence interval (95% CI). Genes found significantly associated with prognosis and pCR across datasets were selected as Core Immune Genes (CIG). Additionally, we added *PDCD1*, which was associated with prognosis and pCR but its gene expression was not available in BrighTNess. Using the CIGs, we evaluated whether combinations of 2 genes enhance their association with each clinical endpoint using different methods: adding, subtracting, multiplying, and ratio. Likelihood ratio testing was used to evaluate the added contribution of each gene in 2 combined cohorts evaluating survival (i.e., CALGB40603 and SCAN-B) and pCR (i.e., CALGB40603 and BrighTNess). Univariate and multivariable Cox regression models and logistic regression models adjusting for treatment arm were used to investigate the association of each combination of 2 CIGs in terms of HRs or ORs with 95% confidence interval (95% CI).

**Table 1. List of interrogated genes**

| **Gene list** | **Interrogated in CALGB40603** | **Interrogated in SCAN-B** | **Interrogated in BrighTNess** |
|---|---|---|---|
| *ABCC11* | yes | yes | yes |
| *ACTG2* | yes | yes | yes |
| *ACTR3B* | yes | yes | yes |
| *AFF3* | yes | yes | yes |
| *AGR2* | yes | yes | yes |
| *AGR3* | yes | yes | yes |
| *ANLN* | yes | yes | yes |
| *AR* | yes | yes | yes |
| *ASPM* | yes | yes | yes |
| *AURKA* | yes | yes | yes |
| *BAG1* | yes | yes | yes |
| *BCL2* | yes | yes | yes |
| *BIRCS* | yes | yes | yes |
| *BLVRA* | yes | yes | yes |
| *BOC* | yes | yes | yes |
| *BRCA1* | yes | yes | yes |
| *BRCA2* | yes | yes | yes |
| *BUB1* | yes | yes | yes |
| *C2orf54* | yes | yes | yes |
| *CCNE1* | yes | yes | yes |
| *CCNB2* | yes | yes | yes |
| *CCND1* | yes | yes | yes |
| *CCNE1* | yes | yes | yes |
| *CD19* | yes | yes | yes |
| *CD2* | yes | yes | yes |
| *CD27* | yes | yes | yes |
| *CD274* | yes | yes | yes |
| *CD3D* | yes | yes | yes |
| *CD3G* | yes | yes | yes |
| *CD4* | yes | yes | yes |
| *CD40* | yes | yes | yes |
| *CD68* | yes | yes | no |
| *CD7* | yes | yes | yes |
| *CD79A* | yes | yes | yes |
| *CD84* | yes | yes | yes |
| *CD86* | yes | yes | yes |
| *CD8A* | yes | yes | yes |
| *CDC20* | yes | yes | yes |
| *CDC6* | yes | yes | yes |
| *CDCA1* | yes | yes | yes |
| *CDCA5* | yes | yes | yes |
| *CDCA8* | yes | yes | yes |
| *CDH3* | yes | yes | yes |
| *CDKN3* | yes | yes | yes |
| *CENPA* | yes | yes | yes |
| *CENPF* | yes | yes | yes |
| *CEP55* | yes | yes | yes |
| *CPUAP1* | yes | yes | yes |
| *CNTNAP2* | yes | yes | no |
| *CREB3L4* | yes | yes | yes |
| *CRYAB* | yes | yes | yes |
| *CTLA4* | yes | yes | yes |
| *CX3CL1* | yes | yes | yes |
| *CXCL13* | yes | yes | yes |
| *CXCL8* | yes | yes | yes |
| *CXCL9* | yes | yes | yes |
| *CXCR6* | yes | yes | yes |
| *CXXC5* | yes | yes | yes |
| *DGKD* | yes | yes | yes |
| *DNAJC12* | yes | yes | yes |
| *DNALI1* | yes | yes | yes |
| *E2F1* | yes | yes | yes |
| *EAF2* | yes | yes | yes |
| *EGFR* | yes | yes | yes |
| *EOMES* | yes | yes | yes |
| *ERBB2* | yes | yes | yes |
| *ERBB3* | yes | yes | yes |
| *ERBB4* | yes | yes | yes |
| *ESR1* | yes | yes | yes |
| *ETFA* | yes | yes | yes |
| *EXO1* | yes | yes | yes |
| *F12* | yes | yes | yes |
| *FA2H* | yes | yes | yes |
| *FGFR1* | yes | yes | yes |
| *FGFR2* | yes | yes | yes |
| *FGFR4* | yes | yes | yes |
| *FHOD1* | yes | yes | yes |
| *FOXA1* | yes | yes | yes |
| *FOXC1* | yes | yes | yes |
| *GABRP* | yes | yes | yes |
| *GARS* | yes | yes | yes |
| *GATA3* | yes | yes | yes |
| *GNLY* | yes | yes | yes |
| *GPNMB* | yes | yes | yes |
| *GPR160* | yes | yes | yes |
| *GRB7* | yes | yes | yes |
| *GSDMB* | yes | yes | yes |
| *GZMA* | yes | yes | yes |
| *GZMB* | yes | yes | yes |
| *HLA.C* | yes | yes | yes |
| *ID4* | yes | yes | yes |
| *IGJ* | yes | yes | yes |
| *IGKC* | yes | no | no |
| *IGL* | no | no | no |
| *IGLV3.25* | yes | no | no |
| *IL18R1* | yes | yes | yes |
| *IL23A* | yes | yes | yes |
| *IL2RG* | yes | yes | yes |
| *IL34* | yes | yes | yes |
| *IRF1* | yes | yes | yes |
| *IRF4* | yes | yes | yes |
| *IRF8* | yes | yes | yes |
| *ISG20* | yes | yes | yes |
| *ITK* | yes | yes | yes |
| *KCTD9* | yes | yes | yes |
| *KIF23* | yes | yes | yes |
| *KIF2C* | yes | yes | yes |
| *KLKS* | yes | yes | yes |
| *KLRB1* | yes | yes | yes |
| *KLRD1* | yes | yes | no |
| *KNTC2* | yes | yes | yes |
| *KRT14* | yes | yes | yes |
| *KRT17* | yes | yes | yes |
| *KRT18* | yes | yes | yes |
| *KRT5* | yes | yes | yes |
| *KRT6B* | yes | yes | yes |
| *KYNU* | yes | yes | yes |
| *LAX1* | yes | yes | yes |
| *LGALS9* | yes | yes | yes |
| *LY9* | yes | yes | yes |
| *MAGED2* | yes | yes | yes |
| *MAPT* | yes | yes | no |
| *MDM2* | yes | yes | yes |
| *MELK* | yes | yes | yes |
| *MFSD2A* | yes | yes | yes |
| *MIA* | yes | yes | yes |
| *MID1* | yes | yes | yes |
| *MKI67* | yes | yes | yes |
| *MLPH* | yes | yes | yes |
| *MMP1* | yes | yes | yes |
| *MMP11* | yes | yes | yes |
| *MND1* | yes | no | yes |
| *MPHOSPH6* | yes | yes | yes |
| *MRAS* | yes | yes | yes |
| *MSLN* | yes | yes | yes |
| *MUCL1* | yes | yes | yes |
| *MYBL2* | yes | yes | yes |
| *MYC* | yes | yes | yes |
| *NAT1* | yes | yes | yes |
| *NDRG2* | yes | yes | yes |
| *NECTIN4* | no | no | yes |
| *NEK2* | yes | yes | yes |
| *NFIB* | yes | yes | yes |
| *NQO1* | yes | yes | yes |
| *NTN3* | yes | yes | yes |
| *ORC6L* | yes | yes | yes |
| *ORMDL3* | yes | yes | yes |
| *PDCD1* | yes | yes | no |
| *PGR* | yes | yes | yes |
| *PHGDH* | yes | yes | yes |
| *PIM2* | yes | yes | yes |
| *PNMT* | yes | yes | yes |
| *POU2AF1* | yes | yes | yes |
| *PSMD3* | yes | yes | yes |
| *PTTG1* | yes | yes | yes |
| *RAD51* | yes | yes | yes |
| *RB1* | yes | yes | yes |
| *RRAGA* | yes | yes | yes |
| RRM2 | yes | yes | yes |
| *S100A9* | yes | yes | yes |
| *SERPINB5* | yes | yes | yes |
| *SFRP1* | yes | yes | yes |
| *SH2D1A* | yes | yes | yes |
| *SIAH2* | yes | yes | yes |
| *SLAMF1* | yes | yes | yes |
| *SLC39A6* | yes | yes | yes |
| *SPDEF* | yes | yes | yes |
| *STARD3* | yes | yes | yes |
| *STAT1* | yes | yes | yes |
| *STAT4* | yes | yes | yes |
| *TCAP* | yes | yes | yes |
| *TFCP2L1* | yes | yes | yes |
| *THSD4* | yes | yes | yes |
| *TMEM45B* | yes | yes | yes |
| *TNFRSF17* | yes | yes | yes |
| *TOP2A* | yes | yes | yes |
| *TROP2* | no | yes | yes |
| *TRPV6* | yes | yes | yes |
| *TSPAN13* | yes | yes | yes |
| *TTK* | yes | yes | yes |
| *TYMS* | yes | yes | yes |
| *UBE2C* | yes | yes | yes |
| *UBE2T* | yes | yes | yes |
| *XBP1* | yes | yes | yes |
| *ZNF552* | yes | yes | yes |

### Example 2. Results

### Example 2.1. Identification of CIGs

To identify genes associated with prognosis, we interrogated the expression of 182 genes in the CALGB40603 (EFS) and 180 genes in the SCAN-B (RFi) datasets. In CALGB40603, high expression of 15 (8.2%) genes, including immune genes such *as IRF4, LAX1, POU2AF1, IGKC* or *CD274,* and low expression of 3 (1.7%) genes was associated with EFS, independently of treatment arm (**Table 2**).

**Table 2. Genes associated with EFS in CALGB40603**

| **gene** | **HR** | **Low 95% CI** | **High 95% CI** | **P** |
|---|---|---|---|---|
| *IRF4* | 0.784 | 0.654 | 0.940 | 0.00860 |
| *XBP1* | 0.785 | 0.647 | 0.952 | 0.01408 |
| *LAX1* | 0.788 | 0.655 | 0.947 | 0.01123 |
| *POU2AF1* | 0.797 | 0.667 | 0.952 | 0.01243 |
| *IGKC* | 0.798 | 0.668 | 0.954 | 0.01321 |
| *CD274* | 0.801 | 0.668 | 0.962 | 0.01736 |
| *KLRD1* | 0.805 | 0.671 | 0.967 | 0.02007 |
| *CD79A* | 0.812 | 0.676 | 0.975 | 0.02532 |
| *IGLV3.25* | 0.816 | 0.677 | 0.984 | 0.03322 |
| *PIM2* | 0.819 | 0.681 | 0.985 | 0.03374 |
| *TNFRSF17* | 0.821 | 0.687 | 0.981 | 0.02968 |
| *GNLY* | 0.824 | 0.685 | 0.993 | 0.04170 |
| *PDCD1* | 0.827 | 0.692 | 0.987 | 0.03558 |
| *CXCR6* | 0.829 | 0.692 | 0.993 | 0.04165 |
| *SLAMF1* | 0.834 | 0.698 | 0.998 | 0.04729 |
| *KRT18* | 1.246 | 1.028 | 1.509 | 0.02479 |
| *TRPV6* | 1.253 | 1.033 | 1.521 | 0.02210 |
| *F12* | 1.398 | 1.149 | 1.700 | 0.00082 |

In SCAN-B, high expression of 37 (20.6%) genes and low expression of 5 (2.8%) genes was associated with RFi (**Table 3**).

**Table 3. Genes associated with RFi in SCAN-B**

| **gene** | **HR** | **Low 95% CI** | **High 95% CI** | **P** |
|---|---|---|---|---|
| *IGJ* | 0.641 | 0.538 | 0.763 | 0.00000 |
| *IRF4* | 0.677 | 0.559 | 0.819 | 0.00006 |
| *TNFRSF17* | 0.682 | 0.569 | 0.817 | 0.00003 |
| *STAT4* | 0.689 | 0.571 | 0.832 | 0.00011 |
| *SLAMF1* | 0.712 | 0.586 | 0.866 | 0.00069 |
| *CD79A* | 0.714 | 0.590 | 0.865 | 0.00058 |
| *NAT1* | 0.716 | 0.560 | 0.915 | 0.00755 |
| *LAX1* | 0.721 | 0.593 | 0.877 | 0.00104 |
| *CXCL13* | 0.722 | 0.602 | 0.866 | 0.00045 |
| *SLC39A6* | 0.723 | 0.567 | 0.922 | 0.00902 |
| *CD274* | 0.733 | 0.603 | 0.891 | 0.00179 |
| *CXCR6* | 0.739 | 0.612 | 0.892 | 0.00159 |
| *PIM2* | 0.739 | 0.601 | 0.908 | 0.00404 |
| *POU2AF1* | 0.746 | 0.623 | 0.895 | 0.00156 |
| *CD3G* | 0.756 | 0.623 | 0.916 | 0.00442 |
| *IL2RG* | 0.762 | 0.623 | 0.931 | 0.00793 |
| *PDCD1* | 0.763 | 0.639 | 0.910 | 0.00267 |
| *CD27* | 0.763 | 0.626 | 0.931 | 0.00760 |
| *IL18R1* | 0.764 | 0.633 | 0.922 | 0.00495 |
| *ITK* | 0.767 | 0.633 | 0.928 | 0.00643 |
| *SH2D1A* | 0.772 | 0.640 | 0.932 | 0.00713 |
| *ESR1* | 0.776 | 0.620 | 0.973 | 0.02785 |
| *CD2* | 0.779 | 0.644 | 0.943 | 0.01035 |
| *CD40* | 0.781 | 0.633 | 0.963 | 0.02091 |
| *LY9* | 0.784 | 0.651 | 0.943 | 0.00986 |
| *KLRB1* | 0.785 | 0.656 | 0.939 | 0.00794 |
| *CD8A* | 0.786 | 0.648 | 0.953 | 0.01454 |
| *GZMA* | 0.789 | 0.651 | 0.955 | 0.01526 |
| *CTLA4* | 0.789 | 0.658 | 0.946 | 0.01059 |
| *CD19* | 0.790 | 0.649 | 0.963 | 0.01933 |
| *CD7* | 0.794 | 0.654 | 0.965 | 0.02013 |
| *EOMES* | 0.796 | 0.657 | 0.963 | 0.01926 |
| *STAT1* | 0.798 | 0.656 | 0.971 | 0.02393 |
| *CD3D* | 0.800 | 0.664 | 0.963 | 0.01856 |
| *IRF1* | 0.807 | 0.665 | 0.978 | 0.02902 |
| *THSD4* | 0.816 | 0.671 | 0.993 | 0.04272 |
| *GZMB* | 0.822 | 0.703 | 0.960 | 0.01355 |
| *FGFR1* | 1.246 | 1.019 | 1.525 | 0.03214 |
| *FHOD1* | 1.292 | 1.053 | 1.585 | 0.01416 |
| *GPNMB* | 1.302 | 1.063 | 1.594 | 0.01066 |
| *MMP11* | 1.354 | 1.097 | 1.673 | 0.00487 |
| *F12* | 1.383 | 1.121 | 1.708 | 0.00252 |

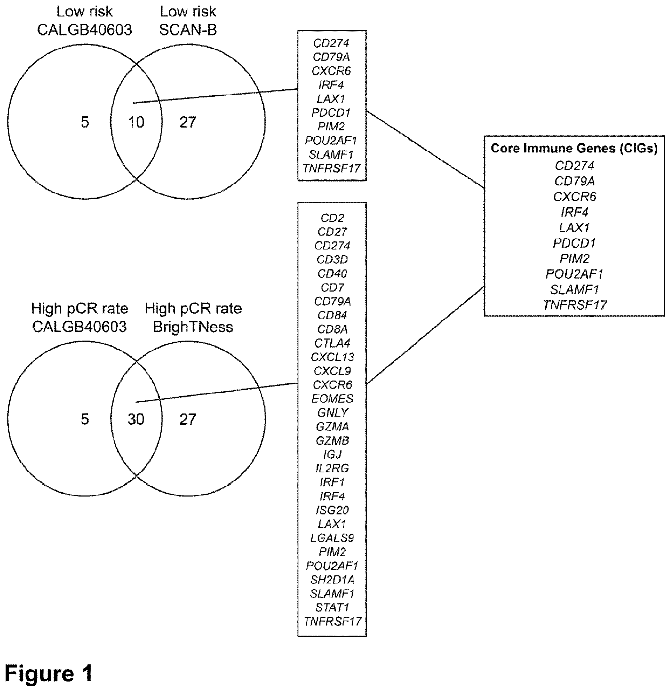

Among the genes associated with low risk, we identified 10 genes that were common for both CALGB40603 and SCAN-B datasets (**Figure 1**).

We then interrogated the expression of 182 genes in the CALGB40603 and 177 genes in the BrighTNess datasets to identify genes associated with pCR. In CALGB40603, high expression of 38 (20.9%) genes, including immune genes such as *CXCR6, PIM2, IGKC, CD274,* or *PDCD1*, and low expression of 9 (5.0%) genes was associated with pCR, independently of treatment arm (**Table 4**).

**Table 4. Genes associated with pCR in CALGB40603**

| **gene** | **HR** | **Low 95% CI** | **High 95% CI** | **P** |
|---|---|---|---|---|
| *CXCR6* | 1.504 | 1.219 | 1.868 | 0.00018 |
| *PIM2* | 1.459 | 1.182 | 1.812 | 0.00051 |
| *IGKC* | 1.452 | 1.178 | 1.803 | 0.00057 |
| *PDCD1* | 1.435 | 1.164 | 1.780 | 0.00085 |
| *CD274* | 1.435 | 1.165 | 1.780 | 0.00082 |
| *TNFRSF17* | 1.434 | 1.162 | 1.783 | 0.00094 |
| *IRF4* | 1.429 | 1.160 | 1.772 | 0.00092 |
| *LAX1* | 1.420 | 1.152 | 1.760 | 0.00114 |
| *CXCL9* | 1.417 | 1.149 | 1.758 | 0.00130 |
| *POU2AF1* | 1.409 | 1.143 | 1.749 | 0.00152 |
| *STAT1* | 1.406 | 1.142 | 1.741 | 0.00150 |
| *GNLY* | 1.402 | 1.139 | 1.736 | 0.00162 |
| *IGLV3.25* | 1.365 | 1.110 | 1.689 | 0.00363 |
| *SLAMF 1* | 1.358 | 1.104 | 1.681 | 0.00424 |
| *KLRD1* | 1.356 | 1.102 | 1.680 | 0.00453 |
| *ISG20* | 1.339 | 1.089 | 1.654 | 0.00610 |
| *CD79A* | 1.338 | 1.088 | 1.654 | 0.00635 |
| *GZMA* | 1.333 | 1.085 | 1.648 | 0.00687 |
| *GZMB* | 1.328 | 1.081 | 1.639 | 0.00759 |
| *IRF1* | 1.327 | 1.081 | 1.638 | 0.00747 |
| *XBP1* | 1.320 | 1.073 | 1.632 | 0.00944 |
| *CD2* | 1.310 | 1.067 | 1.617 | 0.01080 |
| *S100A9* | 1.302 | 1.061 | 1.605 | 0.01232 |
| *CD7* | 1.301 | 1.059 | 1.608 | 0.01325 |
| *CD3D* | 1.295 | 1.055 | 1.599 | 0.01458 |
| *EOMES* | 1.282 | 1.044 | 1.580 | 0.01866 |
| *CD84* | 1.280 | 1.043 | 1.579 | 0.01952 |
| *CD8A* | 1.273 | 1.037 | 1.569 | 0.02226 |
| *IL2RG* | 1.269 | 1.034 | 1.563 | 0.02349 |
| *EAF2* | 1.267 | 1.033 | 1.562 | 0.02434 |
| *CTLA4* | 1.262 | 1.026 | 1.561 | 0.02929 |
| *CD40* | 1.255 | 1.023 | 1.548 | 0.03113 |
| *IGJ* | 1.252 | 1.019 | 1.546 | 0.03395 |
| *CXCL13* | 1.251 | 1.018 | 1.545 | 0.03506 |
| *SH2D1A* | 1.249 | 1.018 | 1.540 | 0.03455 |
| *MDM2* | 1.244 | 1.014 | 1.532 | 0.03808 |
| *LGALS9* | 1.243 | 1.014 | 1.531 | 0.03781 |
| *CD27* | 1.241 | 1.011 | 1.529 | 0.04053 |
| *RB1* | 0.814 | 0.660 | 0.997 | 0.04909 |
| *TMEM45B* | 0.795 | 0.643 | 0.977 | 0.03092 |
| *FGFR2* | 0.781 | 0.630 | 0.962 | 0.02190 |
| *KRT18* | 0.778 | 0.629 | 0.955 | 0.01806 |
| *F12* | 0.764 | 0.620 | 0.937 | 0.01038 |
| *CPUAP1* | 0.764 | 0.618 | 0.938 | 0.01108 |
| *ABCC11* | 0.761 | 0.613 | 0.936 | 0.01094 |
| *FHOD1* | 0.734 | 0.593 | 0.903 | 0.00396 |
| *CCND1* | 0.657 | 0.527 | 0.811 | 0.00013 |

In BrighTNess, high expression of 37 (20.9%) genes including *GZMB, CTLA4, IRF1, CXCL13,* and *CD3D* and low expression of 26 (14.7%) genes was associated with pCR, independently of treatment arm (**Table 5**).

**Table 5. Genes associated with pCR in BrighTNess**

| **gene** | **HR** | **Low 95% CI** | **High 95% CI** | **p** |
|---|---|---|---|---|
| *GZMB* | 1.69 | 1.40 | 2.07 | 0.00000 |
| *CTLA4* | 1.62 | 1.34 | 1.98 | 0.00000 |
| *IRF1* | 1.56 | 1.29 | 1.90 | 0.00001 |
| *CD3D* | 1.55 | 1.28 | 1.89 | 0.00001 |
| *CXCL13* | 1.55 | 1.28 | 1.88 | 0.00001 |
| *STAT1* | 1.54 | 1.27 | 1.87 | 0.00001 |
| *CD2* | 1.53 | 1.27 | 1.87 | 0.00002 |
| *CD274* | 1.52 | 1.26 | 1.85 | 0.00002 |
| *IL2RG* | 1.52 | 1.25 | 1.85 | 0.00002 |
| *CD7* | 1.51 | 1.25 | 1.84 | 0.00002 |
| *CXCL9* | 1.51 | 1.25 | 1.83 | 0.00003 |
| *CD86* | 1.51 | 1.25 | 1.83 | 0.00003 |
| *PIM2* | 1.50 | 1.24 | 1.82 | 0.00003 |
| *CD27* | 1.50 | 1.24 | 1.82 | 0.00004 |
| *CEP55* | 1.49 | 1.24 | 1.82 | 0.00005 |
| *MKI67* | 1.49 | 1.23 | 1.82 | 0.00005 |
| *ITK* | 1.49 | 1.23 | 1.81 | 0.00004 |
| *IRF8* | 1.49 | 1.23 | 1.81 | 0.00005 |
| *SLAMF1* | 1.49 | 1.23 | 1.81 | 0.00004 |
| *CDC6* | 1.48 | 1.23 | 1.81 | 0.00006 |
| *CD3G* | 1.48 | 1.23 | 1.80 | 0.00005 |
| *BIRC5* | 1.48 | 1.23 | 1.80 | 0.00006 |
| *CCNB2* | 1.47 | 1.22 | 1.79 | 0.00008 |
| *KLRB1* | 1.47 | 1.21 | 1.79 | 0.00009 |
| *CDCA5* | 1.47 | 1.21 | 1.78 | 0.00009 |
| *E2F1* | 1.47 | 1.21 | 1.78 | 0.00009 |
| *IL23A* | 1.45 | 1.19 | 1.80 | 0.00038 |
| *CD4* | 1.45 | 1.20 | 1.76 | 0.00014 |
| *GNLY* | 1.45 | 1.20 | 1.76 | 0.00012 |
| *GZMA* | 1.45 | 1.20 | 1.76 | 0.00013 |
| *SH2D1A* | 1.44 | 1.19 | 1.75 | 0.00017 |
| *TYMS* | 1.44 | 1.19 | 1.75 | 0.00017 |
| *KIF2C* | 1.43 | 1.19 | 1.73 | 0.00022 |
| *LAX1* | 1.42 | 1.18 | 1.72 | 0.00027 |
| *CD8A* | 1.42 | 1.18 | 1.72 | 0.00029 |
| *IRF4* | 1.41 | 1.17 | 1.71 | 0.00036 |
| *MELK* | 1.41 | 1.17 | 1.71 | 0.00039 |
| *CXCR6* | 1.41 | 1.17 | 1.71 | 0.00042 |
| *TOP2A* | 1.41 | 1.17 | 1.71 | 0.00046 |
| *CD40* | 1.40 | 1.16 | 1.70 | 0.00054 |
| *POU2AF1* | 1.40 | 1.16 | 1.69 | 0.00046 |
| RRM2 | 1.40 | 1.16 | 1.69 | 0.00055 |
| *KIF23* | 1.39 | 1.16 | 1.69 | 0.00059 |
| *CD84* | 1.39 | 1.15 | 1.68 | 0.00065 |
| *PTTG1* | 1.39 | 1.15 | 1.69 | 0.00069 |
| *BUB1* | 1.39 | 1.15 | 1.68 | 0.00069 |
| *CCNB1* | 1.38 | 1.15 | 1.68 | 0.00078 |
| *KNTC2* | 1.38 | 1.15 | 1.67 | 0.00078 |
| *CD79A* | 1.38 | 1.15 | 1.67 | 0.00078 |
| *ISG20* | 1.38 | 1.14 | 1.67 | 0.00080 |
| *IL18R1* | 1.38 | 1.14 | 1.67 | 0.00104 |
| *STAT4* | 1.37 | 1.14 | 1.66 | 0.00095 |
| *EXO1* | 1.37 | 1.13 | 1.65 | 0.00120 |
| *LGALS9* | 1.36 | 1.13 | 1.65 | 0.00127 |
| *CDCA1* | 1.35 | 1.12 | 1.64 | 0.00162 |
| *LY9* | 1.35 | 1.12 | 1.63 | 0.00193 |
| *RAD51* | 1.35 | 1.12 | 1.63 | 0.00183 |
| *SIAH2* | 1.34 | 1.12 | 1.63 | 0.00217 |
| *ASPM* | 1.34 | 1.12 | 1.63 | 0.00213 |
| *CENPF* | 1.34 | 1.11 | 1.62 | 0.00241 |
| *CDCA8* | 1.34 | 1.11 | 1.62 | 0.00233 |
| *CENPA* | 1.34 | 1.11 | 1.62 | 0.00238 |
| *EOMES* | 1.33 | 1.11 | 1.61 | 0.00255 |
| *NEK2* | 1.33 | 1.11 | 1.61 | 0.00269 |
| *PSMD3* | 1.33 | 1.10 | 1.61 | 0.00345 |
| *BRCA2* | 1.32 | 1.10 | 1.60 | 0.00365 |
| *AURKA* | 1.32 | 1.10 | 1.59 | 0.00378 |
| *CDC20* | 1.31 | 1.09 | 1.58 | 0.00482 |
| *MYBL2* | 1.30 | 1.08 | 1.58 | 0.00532 |
| *UBE2T* | 1.30 | 1.08 | 1.58 | 0.00559 |
| *CCNE1* | 1.29 | 1.07 | 1.57 | 0.00741 |
| *TTK* | 1.29 | 1.07 | 1.56 | 0.00755 |
| *GABRP* | 1.28 | 1.06 | 1.54 | 0.00962 |
| *MND1* | 1.28 | 1.06 | 1.54 | 0.01029 |
| *TNFRSF17* | 1.27 | 1.06 | 1.54 | 0.01064 |
| *UBE2C* | 1.27 | 1.06 | 1.54 | 0.01122 |
| *IGJ* | 1.27 | 1.05 | 1.53 | 0.01253 |
| *CD19* | 1.24 | 1.03 | 1.49 | 0.02440 |
| *CDKN3* | 1.23 | 1.02 | 1.48 | 0.02964 |
| *GRB7* | 0.83 | 0.69 | 1.00 | 0.05000 |
| *ERBB3* | 0.83 | 0.69 | 1.00 | 0.04658 |
| *NTN3* | 0.82 | 0.68 | 0.99 | 0.04234 |
| *EGFR* | 0.82 | 0.67 | 0.99 | 0.03837 |
| *ZNF552* | 0.81 | 0.67 | 0.98 | 0.03142 |
| *AGR3* | 0.81 | 0.67 | 0.98 | 0.03689 |
| *C2orf54* | 0.81 | 0.67 | 0.98 | 0.02924 |
| *ERBB4* | 0.81 | 0.67 | 0.97 | 0.02402 |
| *RB1* | 0.80 | 0.66 | 0.96 | 0.01744 |
| *ID4* | 0.80 | 0.66 | 0.96 | 0.01602 |
| *PNMT* | 0.79 | 0.64 | 0.96 | 0.02067 |
| *TSPAN13* | 0.79 | 0.65 | 0.95 | 0.01320 |
| *FHOD1* | 0.79 | 0.65 | 0.95 | 0.01366 |
| *AGR2* | 0.78 | 0.64 | 0.94 | 0.01121 |
| *AR* | 0.78 | 0.65 | 0.94 | 0.00924 |
| *CCND1* | 0.77 | 0.64 | 0.93 | 0.00633 |
| *SPDEF* | 0.77 | 0.63 | 0.92 | 0.00519 |
| *CRYAB* | 0.76 | 0.63 | 0.92 | 0.00443 |
| *FOXA1* | 0.76 | 0.62 | 0.91 | 0.00370 |
| *MUCL1* | 0.75 | 0.61 | 0.90 | 0.00308 |
| *THSD4* | 0.72 | 0.59 | 0.87 | 0.00071 |
| *NQO1* | 0.70 | 0.58 | 0.85 | 0.00032 |
| *TMEM45B* | 0.70 | 0.56 | 0.86 | 0.00094 |
| *MAGED2* | 0.69 | 0.57 | 0.84 | 0.00024 |
| *MLPH* | 0.69 | 0.57 | 0.84 | 0.00019 |
| *ABCC11* | 0.69 | 0.55 | 0.85 | 0.00074 |

Among the genes whose high expression was associated with pCR, we identified 30 genes that were common for both CALGB40603 and BrighTNess datasets (**Figure 1**).

Since the 10 genes significantly associated with low-risk were also part of the list of 30 genes significantly associated with pCR, except for *PDCD1*, which was not available in BrighTNess but was associated with pCR in CALGB40603, we defined this group of 10 immune genes (i.e., *CD274, CD79A, CXCR6, IRF4, LAX1. PDCD1, PIM2, POU2AF1, SLAMF1*, and *TNFRSF17*) as the CIGs (**Figure 1**).

### Example 2.2. Combination of CIGs to improve the amount of variation explained in prognosis and prediction of pCR

Next, we investigated whether the combination of expression of 2 GICs could improve the amount of variation explained in prognosis, on one hand, and in prediction of pCR, on the other. To do so, we evaluated different combination methodologies such as (1) adding (sum) of the expression of 2 CIGs, (2) subtracting the expression of 2 CIGs, (3) multiplying the expression of 2 CIGs, and (4) dividing (ratio) the expression of 2 CIGs. The amount of variation explained in prognosis and prediction of pCR was defined as the chi-square (χ2) statistic obtained from a likelihood ratio test. Regarding prognosis, the CALB40603 and SCAN-B datasets were combined. Regarding pCR, CALB40603 and BrighTNess were combined.

In general, the sum of the expression of 2 CIGs provided more prognostic information (**Table 6**) and more pCR predictive information (**Table 7**) than a single gene, while subtracting, multiplying, or dividing (i.e., ratio) did not increase the amount of prognostic information (**Table 6**) nor the amount of pCR predictive information (**Table 7**).

**Table 6. Amount of variation explained in prognosis defined by chi-square (x2) statistics from likelihood ratio tests**

| **Combination of 2 CIGs** | **Gene 1** | **Gene 2** | **Sum** | **Substract** | **Multiply** | **Ratio** |
|---|---|---|---|---|---|---|
| *CD79A*_*CD274* | 15.5 | 15.58 | 19.98 | 3.38 | 0 | 2.17 |
| *CD79A*_*CXCR6* | 15.5 | 13.44 | 17.17 | 3.26 | 0.18 | 0.02 |
| *CD79A_LAX1* | 15.5 | 17 | 17.35 | 1.48 | 0.52 | 0.04 |
| *CD79A_PDCD1* | 15.5 | 12.69 | 16.93 | 3.88 | 0.25 | 0.36 |
| *CD79A_PIM2* | 15.5 | 12.7 | 15.51 | 9.46 | 0.09 | 0.66 |
| *CD79A_POU2AF1* | 15.5 | 15.47 | 16.05 | 3.44 | 0.49 | 0.12 |
| *CD79A_SLAMF1* | 15.5 | 14.03 | 16.18 | 7.12 | 0.35 | 1.15 |
| *IRF4_CD274* | 21.67 | 15.58 | 22.54 | 1.77 | 0.01 | 1.9 |
| *CD274_CXCR6* | 15.58 | 13.44 | 16.17 | 0.4 | 0.26 | 0.01 |
| *CD274_LAX1* | 15.58 | 17 | 19.49 | 2.58 | 0.17 | 0.01 |
| *CD274_PDCD1* | 15.58 | 12.69 | 16.35 | 0.11 | 0.11 | 0.09 |
| *CD274_PIM2* | 15.58 | 12.7 | 17.21 | 0.13 | 0.2 | 0.09 |
| *CD274_POU2AF1* | 15.58 | 15.47 | 19.97 | 1.58 | 0.11 | 0.36 |
| *CD274_SLAMF1* | 15.58 | 14.03 | 17.03 | 0.01 | 0.02 | 0.02 |
| *CD274_TNFRSF17* | 15.58 | 19.25 | 23.28 | 7.39 | 0.11 | 7.29 |
| *CXCR6_PDCD1* | 13.44 | 12.69 | 14.44 | 0.08 | 0.03 | 0.43 |
| *CXCR6_PIM2* | 13.44 | 12.7 | 15.18 | 0.88 | 0 | 3.51 |
| *CXCR6_POU2AF1* | 13.44 | 15.47 | 16.94 | 1.06 | 0.03 | 0.25 |
| *CXCR6_SLAMF1* | 13.44 | 14.03 | 14.6 | 0.48 | 0.11 | 0.02 |
| *CXCR6_TNFRSF17* | 13.44 | 19.25 | 20 | 8.27 | 0.72 | 6.56 |
| *LAX1_PDCD1* | 17 | 12.69 | 17.2 | 2.13 | 0.02 | 0.65 |
| *LAX1_POU2AF1* | 17 | 15.47 | 17.2 | 0.05 | 0.28 | 0.13 |
| *LAX1_TNFRSF17* | 17 | 19.25 | 19.81 | 7.99 | 1.39 | 4.92 |
| *PDCD1_PIM2* | 12.69 | 12.7 | 14.96 | 0.48 | 0 | 6 |
| *PDCD1_POU2AF1* | 12.69 | 15.47 | 17.01 | 1.38 | 0.23 | 0.04 |
| *PDCD1_SLAMF1* | 12.69 | 14.03 | 14.76 | 0.1 | 0.06 | 2.16 |
| *PDCD1_TNFRSF17* | 12.69 | 19.25 | 20.37 | 8.02 | 1 | 7.58 |
| *PIM2_SLAMF1* | 12.7 | 14.03 | 14.84 | 0.35 | 0.02 | 0.41 |
| *POU2AF1_SLAMF1* | 15.47 | 14.03 | 16.35 | 3.26 | 0.16 | 1.17 |
| *SLAMF1*_*TNFRSF17* | 14.03 | 19.25 | 19.32 | 12.03 | 1.1 | 7.23 |

**Table 7. Amount of variation explained in prediction of pCR defined by chi-square (x2) statistics from likelihood ratio tests**

| **Combination of 2 CIGs** | **Gene 1** | **Gene 2** | **Sum** | **Substract** | **Multiply** | **Ratio** |
|---|---|---|---|---|---|---|
| *CD79A_SLAMF1* | 15.81 | 17.43 | 17.7 | 5.89 | 0.06 | 4.86 |
| *CD79A_TNFRSF17* | 15.81 | 14.8 | 16.39 | 0.51 | 1.24 | 0.37 |
| *IRF4_CD274* | 21.24 | 25.8 | 28.52 | 1.04 | 0.21 | 0.06 |
| *IRF4_CXCR6* | 21.24 | 22.08 | 25.78 | 1.23 | 2.34 | 0.3 |
| *IRF4_LAX1* | 21.24 | 20.17 | 21.38 | 0.57 | 0.6 | 1.72 |
| *IRF4_SLAMF1* | 21.24 | 17.43 | 21.45 | 5.87 | 0.31 | 1.01 |
| *CD274_CXCR6* | 25.8 | 22.08 | 27.59 | 0 | 2.36 | 0.1 |
| *CD274_LAX1* | 25.8 | 20.17 | 27.04 | 1.76 | 0.34 | 0.01 |
| *CD274_PIM2* | 25.8 | 26.32 | 32.98 | 0.2 | 0 | 1.27 |
| *CD274_POU2AF1* | 25.8 | 18.44 | 28.97 | 0.02 | 0.65 | 2.43 |
| *CXCR6_LAX1* | 22.08 | 20.17 | 24.32 | 2.21 | 2.29 | 0.56 |
| *CXCR6_PIM2* | 22.08 | 26.32 | 29.34 | 0.24 | 1.22 | 1.13 |
| *CXCR6_POU2AF1* | 22.08 | 18.44 | 24.39 | 0.04 | 3.28 | 3.18 |
| *LAX1_POU2AF1* | 20.17 | 18.44 | 20.52 | 3.77 | 0.79 | 0.65 |
| *LAX1_SLAMF1* | 20.17 | 17.43 | 20.52 | 8.03 | 0.36 | 2.89 |
| *POU2AF1_SLAMF1* | 18.44 | 17.43 | 19.9 | 1.7 | 0.57 | 1.84 |
| *SLAMF1*_*TNFRSF17* | 17.43 | 14.8 | 17.59 | 5.45 | 2.15 | 0.29 |

The sum of the expression of 2 CIGs provided more prognostic information than individual CIGs (**Figure 2A**). The sum of the expression of 2 genes provided more pCR predictive information than individual CIGs (**Figure 2B**).

### Example 2.3. Prognostic and predictive value of the combinations of 2 CIGs

Using Cox regression models, we then evaluated the prognostic value of all the possible combinations of 2 CIGs in CALGB40603 and SCAN-B combined. We found that all the combinations (100%) were significantly associated with low risk independently of study and treatment arm (**Table 8**).

**Table 8. Combination of 2 CIGs associated with prognosis in CALGB40603 and SCAN-B**

| **Combination of 2 CIGs** | **HR** | **Low 95% CI** | **High 95% CI** | **p** |
|---|---|---|---|---|
| *CD79A_IRF4* | 0.729 | 0.634 | 0.839 | 0.000010 |
| *CD79A*_*CD274* | 0.693 | 0.592 | 0.811 | 0.000005 |
| *CD79A*_*CXCR6* | 0.740 | 0.643 | 0.851 | 0.000024 |
| *CD79A_LAX1* | 0.758 | 0.666 | 0.862 | 0.000025 |
| *CD79A_PDCD1* | 0.738 | 0.640 | 0.851 | 0.000029 |
| *CD79A_PIM2* | 0.743 | 0.642 | 0.861 | 0.000072 |
| *CD79A_POU2AF1* | 0.773 | 0.683 | 0.874 | 0.000044 |
| *CD79A_SLAMF1* | 0.744 | 0.645 | 0.857 | 0.000045 |
| *CD79A*_*TNFRSF17* | 0.788 | 0.709 | 0.877 | 0.000011 |
| *IRF4_CD274* | 0.617 | 0.506 | 0.751 | 0.000002 |
| *IRF4_CXCR6* | 0.676 | 0.569 | 0.802 | 0.000007 |
| *IRF4_LAX1* | 0.695 | 0.594 | 0.815 | 0.000007 |
| *IRF4_PDCD1* | 0.668 | 0.560 | 0.796 | 0.000007 |
| *IRF4_PIM2* | 0.662 | 0.548 | 0.799 | 0.000018 |
| *IRF4_POU2AF1* | 0.700 | 0.599 | 0.819 | 0.000008 |
| *IRF4_SLAMF1* | 0.667 | 0.558 | 0.798 | 0.000010 |
| *IRF4_TNFRSF17* | 0.736 | 0.648 | 0.836 | 0.000002 |
| *CD274_CXCR6* | 0.686 | 0.572 | 0.823 | 0.000048 |
| *CD274LAX1* | 0.670 | 0.562 | 0.800 | 0.000009 |
| *CD274_PDCD1* | 0.677 | 0.562 | 0.816 | 0.000043 |
| *CD274_PIM2* | 0.640 | 0.519 | 0.790 | 0.000032 |
| *CD274_POU2AF1* | 0.663 | 0.555 | 0.791 | 0.000005 |
| *CD274_SLAMF1* | 0.660 | 0.543 | 0.802 | 0.000030 |
| *CD274_TNFRSF17* | 0.697 | 0.603 | 0.805 | 0.000001 |
| *CXCR6_LAX1* | 0.719 | 0.616 | 0.839 | 0.000029 |
| *CXCR6_PDCD1* | 0.723 | 0.613 | 0.853 | 0.000115 |
| *CXCR6_PIM2* | 0.692 | 0.576 | 0.831 | 0.000085 |
| *CXCR6_POU2AF1* | 0.718 | 0.616 | 0.838 | 0.000025 |
| *CXCR6_SLAMF1* | 0.715 | 0.604 | 0.847 | 0.000100 |
| *CXCR6_TNFRSF17* | 0.742 | 0.653 | 0.843 | 0.000005 |
| *LAX1_PDCD1* | 0.711 | 0.606 | 0.834 | 0.000027 |
| *LAX1_PIM2* | 0.707 | 0.597 | 0.837 | 0.000058 |
| *LAX1_POU2AF1* | 0.738 | 0.641 | 0.850 | 0.000026 |
| *LAX1_SLAMF1* | 0.712 | 0.606 | 0.836 | 0.000036 |
| *LAX1*_*TNFRSF17* | 0.761 | 0.676 | 0.857 | 0.000006 |
| *PDCD1_PIM2* | 0.689 | 0.572 | 0.831 | 0.000095 |
| *PDCD1_POU2AF1* | 0.712 | 0.607 | 0.834 | 0.000026 |
| *PDCD1_SLAMF1* | 0.706 | 0.593 | 0.841 | 0.000097 |
| *PDCD1*_*TNFRSF17* | 0.734 | 0.644 | 0.837 | 0.000004 |
| *PIM2_POU2AF1* | 0.721 | 0.613 | 0.849 | 0.000083 |
| *PIM2_SLAMF1* | 0.684 | 0.564 | 0.829 | 0.000107 |
| *PIM2*_*TNFRSF17* | 0.746 | 0.653 | 0.852 | 0.000015 |
| *POU2AF1_SLAMF1* | 0.716 | 0.610 | 0.839 | 0.000037 |
| *POU2AF1_TNFRSF17* | 0.774 | 0.691 | 0.868 | 0.000011 |
| *SLAMF1*_*TNFRSF17* | 0.742 | 0.651 | 0.845 | 0.000007 |

Additionally, we compared head-to-head the Cox regression models using an individual gene vs the combination of 2 CIGs. Generally, the sum of the expression of 2 CIGs provided more prognostic information than a single gene as shown by higher likelihood ratios in the model of the combination of 2 CIGs (**Table 9**).

**Table 9. Head to head comparison of Cox regression models of an individual gene vs the combination of 2 CIGs**

| **Combination of 2 CIGs** | **cox_model_gene1.vs.cox_model_2CIGs_sum** | | | **cox_model_gene2.vs.cox_model_2CIGs _sum** | | |
|---|---|---|---|---|---|---|
| | **Loglik gene1** | **Loglik Sum** | **χ2** | **Loglik gene2** | **Loglik Sum** | **χ2** |
| *CD79A_IRF4* | -1375.79 | -1372.69 | 6.2 | -1372.7 | -1372.69 | 0.03 |
| *CD79A_CD274* | -137579 | -1373.34 | 4.89 | -1375.75 | -1373.34 | 4.82 |
| *CD79A_CXCR6* | -1375.79 | -1374.95 | 1.67 | -1376.82 | -1374.95 | 3.73 |
| *CD79A_LAX1* | -137579 | -1374.74 | 2.09 | -1375.04 | -1374.74 | 0.59 |
| *CD79A_PDCD1* | -1375.79 | -1375.06 | 1.45 | -1377.19 | -1375.06 | 4.27 |
| *CD79A_PIM2* | -1375.79 | -1375.7 | 0.17 | -1377.19 | -1375.7 | 2.97 |
| *CD79A_POU2AF1* | -1375.79 | -1375.51 | 0.55 | -1375.81 | -1375.51 | 0.58 |
| *CD79A_SLAMF1* | -1375.79 | -1375.45 | 0.68 | -1376.53 | -1375.45 | 2.16 |
| *CD79A_TNFRSF17* | -1375.79 | -1373.9 | 3.77 | -1373.92 | -1373.9 | 0.03 |
| *IRF4_CD274* | -1372.7 | -1372 | 1.42 | -1375.75 | -1372 | 7.51 |
| *IRF4_CXCR6* | -1372.70 | -1372.68 | 0.01 | -1376.82 | -1372.7 | 8.24 |
| *IRF4_LAX1* | -1372.70 | -1372.64 | 0.12 | -1375.04 | -1372.64 | 4.79 |
| *IRF4_PDCD1* | -1372.70 | -1372.65 | 0.11 | -1377.19 | -1372.65 | 9.09 |
| *IRF4_PIM2* | -1372.70 | -1372.37 | 0.67 | -1377.19 | -1372.37 | 9.64 |
| *IRF4_POU2AF1* | -1372.70 | -1372.66 | 0.02 | -1375.81 | -1372.7 | 6.22 |
| *IRF4_SLAMF1* | -1372.7 | -1372.59 | 0.23 | -1376.53 | -1372.59 | 7.87 |
| *IRF4_TNFRSF17* | -1372.7 | -1372.39 | 0.64 | -1373.92 | -1372.39 | 306 |
| *CD274_CXCR6* | -1375.75 | -1375.3 | 0.9 | -1376.82 | -1375.3 | 3.04 |
| *CD274_LAX1* | -1375.75 | -1373.79 | 3.92 | -1375.04 | -1373.79 | 2.49 |
| *CD274_PDCD1* | -1375.75 | -1375.16 | 1.19 | -1377.19 | -1375.16 | 4.08 |
| *CD274_PIM2* | -1375.75 | -1374.83 | 1.84 | -1377.19 | -1374.83 | 4.72 |
| *CD274_POU2AF1* | -1375.75 | -1373.46 | 4.58 | -1375.81 | -1373.46 | 4.69 |
| *CD274_SLAMF1* | -1375.75 | -1374.97 | 1.57 | -1376.53 | -1374.97 | 3.12 |
| *CD274_TNFRSF17* | -1375.75 | -1371.79 | 7.93 | -1373.92 | -1371.79 | 4.26 |
| *CXCR6_LAX1* | -1376.82 | -1374.84 | 3.96 | -1375.04 | -1374.84 | 0.4 |
| *CXCR6_PDCD1* | -137682 | -1376.31 | 1.03 | -1377.19 | -1376.31 | 1.78 |
| *CXCR6_PIM2* | -1376.82 | -1375.95 | 1.73 | -1377.19 | -1375.95 | 2.48 |
| *CXCR6_POU2AF1* | -1376.82 | -1375.04 | 3.56 | -1375.81 | -1375.04 | 1.53 |
| *CXCR6_SLAMF1* | -137682 | -1376.22 | 1.2 | -1376.53 | -1376.22 | 0.61 |
| *CXCR6_TNFRSF17* | -1376.82 | -1373.45 | 6.74 | -1373.92 | -1373.45 | 0.94 |
| *LAX1_PDCD1* | -137504 | -1374.71 | 0.65 | -1377.19 | -1374.71 | 4.96 |
| *LAX1_POU2AF1* | -137504 | -1374.85 | 0.38 | -1375.81 | -1374.85 | 1.91 |
| *LAX1_SLAMF1* | -1375.04 | -1374.98 | 0.11 | -1376.53 | -1374.98 | 3.09 |
| *LAX1_TNFRSF17* | -1375.04 | -1373.61 | 2.86 | -1373.92 | -1373.61 | 0.61 |
| *PDCD1_PIM2* | -1377.19 | -1376.05 | 2.29 | -1377.19 | -1376.05 | 2.28 |
| *PDCD1_POU2AF1* | -1377.19 | -1374.98 | 4.43 | -1375.81 | -1374.98 | 1.65 |
| *PDCD1_SLAMF1* | -1377.19 | -1376.09 | 2.21 | -1376.53 | -1376.09 | 0.87 |
| *PDCD1_TNFRSF17* | -1377.19 | -1373.27 | 7.84 | -1373.92 | -1373.27 | 1.28 |
| *PIM2_POU2AF1* | -1377.19 | -1375.78 | 2.83 | -1375.81 | -1375.78 | 0.06 |
| *PIM2_SLAMF1* | -1377.19 | -1376.09 | 2.21 | -1376.53 | -1376.09 | 0.88 |
| *PIM2_TNFRSF17* | -1377.19 | -1373.91 | 6.56 | -1373.92 | -1373.91 | 0.01 |
| *POU2AF1_SLAMF1* | -1375.81 | -1375.35 | 0.91 | -1376.53 | -1375.35 | 2.35 |
| *SLAMF1_TNFRSF17* | -1376.53 | -1373.75 | 5.54 | -1373.92 | -1373.75 | 0.32 |

Next, we used logistic regression models to evaluate the association of all the possible combinations of 2 CIGs in CALGB40603 and BrighTNess combined. We found that all the combinations (100%) were significantly associated with pCR independently of study and treatment arm (**Table 10**).

**Table 10. Combination of 2 CIGs associated with pCR prediction in CALGB40603 and BrighTNess**

| **Combination of 2 CIGs** | **HR** | **Low 95% CI** | **High 95% CI** | **P** |
|---|---|---|---|---|
| *CD79A_IRF4* | 1.392 | 1.199 | 1.621 | 0.00002 |
| *CD79A*_*CD274* | 1.622 | 1.341 | 1.968 | 0.00000 |
| *CD79A*_*CXCR6* | 1.518 | 1.273 | 1.819 | 0.00000 |
| *CD79A_LAX1* | 1.379 | 1.193 | 1.598 | 0.00002 |
| *CD79A_PIM2* | 1.468 | 1.244 | 1.737 | 0.00001 |
| *CD79A_POU2AF1* | 1.387 | 1.189 | 1.623 | 0.00004 |
| *CD79A_SLAMF1* | 1.431 | 1.21 | 1.699 | 0.00004 |
| *CD79A*_*TNFRSF17* | 1.297 | 1.142 | 1.476 | 0.00007 |
| *IRF4_CD274* | 1.76 | 1.426 | 2.181 | 0.00000 |
| *IRF4_CXCR6* | 1.669 | 1.366 | 2.05 | 0.00000 |
| *IRF4_LAX1* | 1.452 | 1.238 | 1.708 | 0.00001 |
| *IRF4_PIM2* | 1.599 | 1.326 | 1.935 | 0.00000 |
| *IRF4_POU2AF1* | 1.504 | 1.261 | 1.8 | 0.00001 |
| *IRF4_SLAMF1* | 1.577 | 1.298 | 1.924 | 0.00001 |
| *IRF4_TNFRSF17* | 1.36 | 1.182 | 1.571 | 0.00002 |
| *CD274_CXCR6* | 1.839 | 1.46 | 2.331 | 0.00000 |
| *CD274_LAX1* | 1.678 | 1.377 | 2.053 | 0.00000 |
| *CD274_PIM2* | 2.006 | 1.576 | 2.566 | 0.00000 |
| *CD274_POU2AF1* | 1.871 | 1.485 | 2.372 | 0.00000 |
| *CD274_SLAMF1* | 1.821 | 1.437 | 2.322 | 0.00000 |
| *CD274_TNFRSF17* | 1.554 | 1.302 | 1.865 | 0.00000 |
| *CXCR6_LAX1* | 1.595 | 1.322 | 1.934 | 0.00000 |
| *CXCR6_PIM2* | 1.862 | 1.482 | 2.355 | 0.00000 |
| *CXCR6_POU2AF1* | 1.699 | 1.372 | 2.116 | 0.00000 |
| *CXCR6_SLAMF1* | 1.676 | 1.344 | 2.103 | 0.00001 |
| *CXCR6_TNFRSF17* | 1.451 | 1.233 | 1.716 | 0.00001 |
| *LAX1_PIM2* | 1.56 | 1.304 | 1.872 | 0.00000 |
| *LAX1_POU2AF1* | 1.471 | 1.243 | 1.747 | 0.00001 |
| *LAX1_SLAMF1* | 1.522 | 1.267 | 1.836 | 0.00001 |
| *LAX1*_*TNFRSF17* | 1.348 | 1.176 | 1.551 | 0.00002 |
| *PIM2_POU2AF1* | 1.619 | 1.329 | 1.982 | 0.00000 |
| *PIM2_SLAMF1* | 1.745 | 1.396 | 2.192 | 0.00000 |
| *PIM2*_*TNFRSF17* | 1.425 | 1.22 | 1.67 | 0.00001 |
| *POU2AF1_SLAMF1* | 1.591 | 1.295 | 1.965 | 0.00001 |
| *POU2AF1_TNFRSF17* | 1.349 | 1.168 | 1.564 | 0.00006 |
| *SLAMF1*_*TNFRSF17* | 1.401 | 1.195 | 1.649 | 0.00004 |

Finally, we did a head-to-head comparison of the logistic regression models using an individual gene vs the combination of 2 CIGs. Generally, the sum of the expression of 2 CIGs provided more prognostic information than a single gene as shown by lower residual deviation in the model of the combination of 2 CIGs (**Table 11**).

**Table 11. Head-to-head comparison of Cox regression models of an individual gene vs the combination of 2 CIGs**

| **Combination of 2 CIGs** | **glm_model_gene1.vs.glmmodel_2CIGs_sum** | | | **glm_model_gene2.vs.glm_model_2CIGs_sum** | | |
|---|---|---|---|---|---|---|
| | **Resid.Dev. genel** | **Resid.Dev. Sum** | **Deviance** | **Resid.Dev. gene2** | **Resid.Dev. Sum** | **Deviance** |
| *CD79A_IRF4* | 1190.7 | 1185.19 | 5.52 | 1185.27 | 1185.19 | 0.09 |
| *CD79A_CD274* | 1190.7 | 1177.6 | 13.1 | 1180.71 | 1177.6 | 3.11 |
| *CD79A_CXCR6* | 1190.7 | 1182.77 | 7.93 | 1184.43 | 1182.77 | 1.66 |
| *CD79A_LAX1* | 1190.7 | 1186.28 | 4.42 | 1186.34 | 1186.28 | 0.06 |
| *CD79A_PIM2* | 1190.7 | 1179.53 | 11.18 | 1180.19 | 1179.53 | 0.66 |
| *CD79A_SLAMF1* | 1190.7 | 1188.39 | 2.31 | 1189.08 | 1188.39 | 0.69 |
| *CD79A*_*TNFRSF17* | 1190.7 | 1190.03 | 0.68 | 1191.71 | 1190.03 | 1.68 |
| *IRF4_CD274* | 1185.27 | 1177.16 | 8.12 | 1180.71 | 1177.16 | 3.56 |
| *IRF4_CXCR6* | 1185.27 | 1180.6 | 4.67 | 1184.43 | 1180.6 | 3.83 |
| *IRF4_LAX1* | 1185.27 | 1185 | 0.28 | 1186.34 | 1185 | 1.34 |
| *IRF4_POU2AF1* | 1185.27 | 1185.13 | 014 | 1188.07 | 1185.13 | 2.94 |
| *IRF4_SLAMF1* | 1185.27 | 1184.78 | 0.49 | 1189.08 | 1184.78 | 4.3 |
| *IRF4_TNFRSF17* | 1185.27 | 1185.26 | 0.01 | 1191.71 | 1185.26 | 6.45 |
| *CD274_CXCR6* | 1180.71 | 1178.58 | 2.14 | 1184.43 | 1178.58 | 5.86 |
| *CD274_LAX1* | 1180.71 | 1178.14 | 2.58 | 1186.34 | 1178.14 | 8.21 |
| *CD274_PIM2* | 1180.71 | 1173.53 | 7.19 | 1180.19 | 1173.53 | 6.66 |
| *CD274_POU2AF1* | 1180.71 | 1176.36 | 4.35 | 1188.07 | 1176.36 | 11.71 |
| *CD274_SLAMF1* | 1180.71 | 1179.91 | 0.81 | 1189.08 | 1179.91 | 9.17 |
| *CD274*_*TNFRSF17* | 1180.71 | 1177.44 | 3.28 | 1191.71 | 1177.44 | 14.27 |
| *CXCR6_LAX1* | 1184.43 | 1181.85 | 2.59 | 1186.34 | 1181.85 | 4.49 |
| *CXCR6_PIM2* | 1184.43 | 1176.91 | 7.52 | 1180.19 | 1176.91 | 3.28 |
| *CXCR6_POU2AF1* | 1184.43 | 1181.73 | 2.71 | 1188.07 | 1181.73 | 6.35 |
| *CXCR6_SLAMF1* | 1184.43 | 1184.16 | 0.28 | 1189.08 | 1184.16 | 4.92 |
| *CXCR6_TNFRSF17* | 1184.43 | 1183.3 | 1.13 | 1191.71 | 1183.3 | 8.4 |
| *LAX1_POU2AF1* | 1186.34 | 1185.89 | 0.45 | 1188.07 | 1185.89 | 218 |
| *LAX1_SLAMF1* | 1186.34 | 1185.87 | 0.47 | 1189.08 | 1185.87 | 3.21 |
| *LAX1_TNFRSF17* | 1186.34 | 1186.27 | 0.08 | 1191.71 | 1186.27 | 5.44 |
| *PIM2_POU2AF1* | 1180.19 | 1179.92 | 0.27 | 1188.07 | 1179.92 | 8.15 |
| *PIM2_SLAMF1* | 1180.19 | 1180.09 | 0.1 | 1189.08 | 1180.09 | 8.99 |
| *PIM2*_*TNFRSF17* | 1180.19 | 1179.97 | 0.22 | 1191.71 | 1179.97 | 11.73 |
| *POU2AF1_SLAMF1* | 1188.07 | 1186.61 | 1.46 | 1189.08 | 1186.61 | 2.47 |
| *POU2AF1_TNFRSF1 7* | 1188.07 | 1188.08 | 0.01 | 1191.71 | 1188.07 | 3.64 |
| *SLAMF1*_*TNFRSF17* | 1189.08 | 1188.04 | 1.04 | 1191.71 | 1188.04 | 3.67 |

## Claims

1. *In vitro* method for identifying biomarker signatures for the prognosis of patients suffering from triple-negative breast cancer, which comprises:
a. Measuring the level of expression of at least two genes selected from the group consisting of: [*IRF4, LAX1, POU2AF1, CD274, CD79A, TNFRSF17, PIM2, PDCD1, CXCR6* and/or *SLAMF1],* in a biological sample obtained from the patient;
b. Determining a combination score value by calculating the sum of the expression of the two genes; and
c. Wherein if a deviation of the combination score value is identified, as compared with a pre-established reference value, this is indicative that the biomarker signature may be used for the prognosis of patients suffering from triple-negative breast cancer.

2. *In vitro* method for the prognosis of patients suffering from triple-negative breast cancer, according to claim 1, which comprises:
a. Measuring the level of expression of at least two genes selected from the group consisting of: [*IRF4, LAX1, POU2AF1, CD274, CD79A, TNFRSF17, PIM2, PDCD1, CXCR6* and/or *SLAMF1*]*,* in a biological sample obtained from the patient;
b. Determining a combination score value by calculating the sum of the expression of the two genes; and
c. Wherein if a deviation of the combination score value is identified, as compared with a pre-established reference value, this is indicative of the prognosis of patients suffering from triple-negative breast cancer.

3. *In vitro* method, according to claim 2, for the prognosis of patients suffering from triple-negative breast cancer, which comprises:
a. Measuring the level of expression of at least one of the combinations of two genes of **Table 6** or **Table 7** in a biological sample obtained from the patient;
b. Determining a combination score value by calculating the sum of the expression of the two genes; and
c. Wherein if a deviation of the combination score value is identified, as compared with a pre-established reference value, this is indicative of the prognosis of patients suffering from triple-negative breast cancer.

4. *In vitro* method for the prognosis of patients suffering from triple-negative breast cancer, according to claims 2 or 3, which comprises:
a. Measuring the level of expression of at least two genes selected from the group consisting of: [*IRF4, LAX1, POU2AF1, CD274, CD79A, TNFRSF17, PIM2, PDCD1, CXCR6* and/or *SLAMF1*]*,* in a biological sample obtained from the patient;
b. Determining a combination score value by calculating the sum of the expression of the two genes; and
c. Wherein if an increased score value is identified, as compared with a pre-established reference value, this is indicative of good prognosis.

5. *In vitro* method for the prognosis of patients suffering from triple-negative breast cancer, according to any of the claims 2 to 4, which comprises:
a. Measuring the level of expression of at least one of the combinations of two genes of **Table 6** or **Table 7** in a biological sample obtained from the patient;
b. Determining a combination score value by calculating the sum of the expression of the two genes; and
c. Wherein if an increased score value is identified, as compared with a pre-established reference value, this is indicative of good prognosis.

6. *In vitro* method for the prognosis of patients suffering from triple-negative breast cancer, according to any of the claims 2 to 5, which comprises:
a. Measuring the level of expression of at least one of the combinations of two genes of **Table 6** in a biological sample obtained from the patient;
b. Determining a combination score value by calculating the sum of the expression of the two genes; and
c. Wherein if an increased score value is identified, as compared with a pre-established reference value, this is indicative of lack of cancer recurrence.

7. *In vitro* method for the prognosis of patients suffering from triple-negative breast cancer, according to any of the claims 2 to 6, which comprises:
a. Measuring the level of expression of at least one of the combinations of two genes of **Table 7** in a biological sample obtained from the patient;
b. Determining a combination score value by calculating the sum of the expression of the two genes; and
c. Wherein if an increased score value is identified, as compared with a pre-established reference value, this is indicative of response to chemotherapy-based therapy.

8. *In vitro* use of at least two genes selected from the group consisting of: [*IRF4, LAX1, POU2AF1, CD274, CD79A, TNFRSF17, PIM2, PDCD1, CXCR6* and/or *SLAMF1*], or of a kit comprising reagents for the assessing the level of expression of the genes, for identifying biomarker signatures for the prognosis of patients suffering from triple-negative breast cancer.

9. *In vitro* use, according to claim 8, at least two genes selected from the group consisting of: [*IRF4, LAX1, POU2AF1, CD274, CD79A, TNFRSF17, PIM2, PDCD1, CXCR6* and/or *SLAMF*], or of a kit comprising reagents for the assessing the level of expression of the genes, for the prognosis of patients suffering from triple-negative breast cancer.

10. *In vitro* use, according to claim 9, of at least one of the combinations of two genes of **Table 6** or **Table 7,** or of a kit comprising reagents for the assessing the level of expression of the genes, for the prognosis of patients suffering from triple-negative breast cancer.

11. *In vitro* use, according to any of the claims 9 or 10, of at least one of the combinations of two genes of **Table 6,** or of a kit comprising reagents for the assessing the level of expression of the genes, for predicting recurrence.

12. *In vitro* use, according to any of the claims 9 to 11, of at least one of the combinations of two genes of **Table 7,** or of a kit comprising reagents for the assessing the level of expression of the genes, for predicting response to chemotherapy-based therapy.

13. Chemotherapy, PARP1 inhibitors, antibody drug conjugates and PD-1 or PD-L1 inhibitors for use in the treatment of patients suffering from triple-negative breast cancer, wherein the method comprises assessing the prognosis of the patients suffering from triple-negative breast cancer, by following the method of claims 2 to 7.

14. Chemotherapy, PARP1 inhibitors, antibody drug conjugates and PD-1 or PD-L1 inhibitor for use, according to claim 13, selected from the list comprising: Paclitaxel, Nab-paclitaxel, Docetaxel, Carboplatin, Cisplatinum, Cyclophosphamide, Doxorubicin, Olaparib, Niraparib, Talazoparib, Nivolumab, Pembrolizumab, Atezolizumab, Avelumab, Durvalumab, Cemiplimab, Tislelizumab, Dostarlimab or Retifanlimab, trastuzumab deruxtecan, sacitumab govitecan and patritumab deruxtecan.
